# EUROPEAN PATENT APPLICATION

(11) **EP 4 230 608 A1**
(43) Date of publication of application: **23.08.2023**
(21) Application number: 21880013.4
(22) Date of filing: 08.10.2021
(51) Int. Cl.: C07C 21/18, C07C 17/38, B65D 85/84

(54) **METHOD FOR STORING FLUORO-2-BUTENE**

(30) Priority: 15.10.2020 JP 2020173921
(71) Applicant: Resonac Corporation, Tokyo 105-8518 (JP)
(72) Inventor: SUZUKI Atsushi, Tokyo 105-8518 (JP)
(74) Representative: Strehl Schübel-Hopf & Partner
(86) International application number: PCT/JP2021/037428
(87) International publication number: WO 2022/080274

(57) **Abstract**

Provided is a method for storing a fluoro-2-butene by which isomerization reaction is unlikely to proceed during storage. A fluoro-2-butene represented by general formula C₄HₓF_{y} where x is 0 or more and 7 or less, y is 1 or more and 8 or less, and x + y is 8 contains or does not contain at least one of chromium, molybdenum, iron, zinc, and aluminum as a metal impurity. The fluoro-2-butene is stored in a container in which the total concentration of chromium, molybdenum, iron, zinc, and aluminum is 1,000 ppb by mass or less when containing at least one of chromium, molybdenum, iron, zinc, and aluminum.

## Description

### Technical Field

The present invention relates to a method for storing a fluoro-2-butene.

### Background Art

Unsaturated fluorocarbons disclosed, for example, in PTLs 1 and 2 may be used as an etching gas for dry etching. Of the unsaturated fluorocarbons, a fluoro-2-butene has attracted attention as an etching gas usable in state-of-the-art dry etching processes.

### Citation List

### Patent Literature

PTL 1: JP 6451810 B
PTL 2: JP 2019-034972 A

### Summary of Invention

### Technical Problem

Fluoro-2-butenes, however, have Z- and E-geometric isomers, and isomerization reaction may proceed during storage for a long time.

The present invention is intended to provide a method for storing a fluoro-2-butene by which isomerization reaction is unlikely to proceed during storage.

### Solution to Problem

To solve the problems, aspects of the present invention are the following [1] to [3].
[1] A method for storing a fluoro-2-butene represented by general formula C₄HₓF_{y} where x is 0 or more and 7 or less, y is 1 or more and 8 or less, and x + y is 8, in which
   the fluoro-2-butene contains or does not contain at least one of chromium, molybdenum, iron, zinc, and aluminum as a metal impurity, and the fluoro-2-butene is stored in a container in which the total concentration of chromium, molybdenum, iron, zinc, and aluminum is 1,000 ppb by mass or less when the fluoro-2-butene contains at least one of chromium, molybdenum, iron, zinc, and aluminum.
[2] The method for storing a fluoro-2-butene according to the aspect [1], in which the fluoro-2-butene is at least one selected from (Z)-1,1,1,4,4,4-hexafluoro-2-butene, (E)-1,1,1,4,4,4-hexafluoro-2-butene, (Z)-1,1,1,2,4,4,4-heptafluoro-2-butene, (E)-1,1,1,2,4,4,4-heptafluoro-2-butene, (Z)-1,1,1,2,3,4,4,4-octafluoro-2-butene, and (E)-1,1,1,2,3,4,4,4-octafluoro-2-butene.
[3] The method for storing a fluoro-2-butene according to the aspect [1] or [2], in which the fluoro-2-butene is stored at a temperature of -20°C or more and 50°C or less.

### Advantageous Effects of Invention

According to the present invention, isomerization reaction of a fluoro-2-butene is unlikely to proceed during storage.

### Description of Embodiments

Embodiments of the present invention will now be described. The embodiments are merely examples of the present invention, and the present invention is not limited to the embodiments. Various modifications or improvements can be made in the embodiments, and such modifications and improvements can be encompassed by the present invention.

The method for storing a fluoro-2-butene pertaining to the present embodiment is a method for storing a fluoro-2-butene represented by general formula C₄HₓF_{y} where x is 0 or more and 7 or less, y is 1 or more and 8 or less, and x + y is 8. In the method, the fluoro-2-butene contains or does not contain at least one of chromium (Cr), molybdenum (Mo), iron (Fe), zinc (Zn), and aluminum (Al) as a metal impurity, and the fluoro-2-butene is stored in a container in which the total concentration of chromium, molybdenum, iron, zinc, and aluminum is 1,000 ppb by mass or less when the fluoro-2-butene contains at least one of chromium, molybdenum, iron, zinc, and aluminum.

When a fluoro-2-butene contains at least one of chromium, molybdenum, iron, zinc, and aluminum as a metal impurity, the catalytic action of the metal impurity accelerates isomerization reaction of the fluoro-2-butene. Hence, a fluoro-2-butene containing a metal impurity may be isomerized during storage, and the purity may decrease.

A fluoro-2-butene stored by the method for storing a fluoro-2-butene pertaining to the present embodiment contains no metal impurity or contains a metal impurity at a small content and thus is unlikely to be isomerized even when stored for a long time, and the purity is unlikely to decrease. Accordingly, the fluoro-2-butene can be stably stored over a long time.

The technology disclosed in PTLs 1 and 2 does not consider the concentration of a metal impurity in an unsaturated fluorocarbon. Hence, when a fluoro-2-butene is stored by the technology disclosed in PTLs 1 and 2, a metal impurity may accelerate isomerization reaction of the fluoro-2-butene. As a result, the fluoro-2-butene may be isomerized during storage, and the purity may decrease.

Hereinafter, the method for storing a fluoro-2-butene pertaining to the present embodiment will be described in further detail.

### [Fluoro-2-butene]

The fluoro-2-butene pertaining to the present embodiment is represented by general formula C₄HₓF_{y} and satisfies three requirements in the general formula: x is 0 or more and 7 or less; y is 1 or more and 8 or less; and x + y is 8. The fluoro-2-butene may be any type that satisfies the above requirements.

Specific examples of the fluoro-2-butene include (Z)-CHF₂-CF=CF-CF₃, (E)-CHF₂-CF=CF-CF₃, (Z)-CF₃-CH=CF-CF₃, (E)-CF₃-CH=CF-CF₃, (Z)-CH₂F-CF=CF-CF₃, (E)-CH₂F-CF=CF-CF₃, (Z)-CHF₂-CH=CF-CF₃, (E)-CHF₂-CH=CF-CF₃, (Z)-CHF₂-CF=CF-CHF₂, (E)-CHF₂-CF=CF-CHF₂, (Z)-CF₃-CH=CH-CF₃, (E)-CF₃-CH=CH-CF₃, (Z)-CH₃-CF=CF-CF₃, (E)-CH₃-CF=CF-CF₃, (Z)-CH₂F-CH=CF-CF₃, (E)-CH₂F-CH=CF-CF₃, (Z)-CH₂F-CF=CH-CF₃, (E)-CH₂F-CF=CH-CF₃, (Z)-CH₂F-CF=CF-CHF₂, (E)-CH₂F-CF=CF-CHF₂, (Z)-CHF₂-CH=CH-CF₃, (E)-CHF₂-CH=CH-CF₃, (Z)-CHF₂-CF=CH-CHF₂, (E)-CHF₂-CF=CH-CHF₂, (Z)-CH₃-CH=CF-CF₃, (E)-CH₃-CH=CF-CF₃, (Z)-CH₃-CF=CH-CF₃, (E)-CH₃-CF=CH-CF₃, (Z)-CH₃-CF=CF-CHF₂, (E)-CH₃-CF=CF-CHF₂, (Z)-CH₂F-CH=CH-CF₃, (E)-CH₂F-CH=CH-CF₃, (Z)-CH₂F-CH=CF-CHF₂, (E)-CH₂F-CH=CF-CHF₂, (Z)-CH₂F-CF=CH-CHF₂, (E)-CH₂F-CF=CH-CHF₂, (Z)-CH₂F-CF=CF-CH₂F, (E)-CH₂F-CF=CF-CH₂F, (Z)-CHF₂-CH=CH-CHF₂, (E)-CHF₂-CH=CH-CHF₂, (Z)-CH₃-CH=CH-CF₃, (E)-CH₃-CH=CH-CF₃, (Z)-CH₃-CH=CF-CHF₂, (E)-CH₃-CH=CF-CHF₂, (Z)-CH₃-CF=CH-CHF₂, (E)-CH₃-CF=CH-CHF₂, (Z)-CH₃-CF=CF-CH₂F, (E)-CH₃-CF=CF-CH₂F, (Z)-CH₂F-CF=CH-CH₂F, (E)-CH₂F-CF=CH-CH₂F, (Z)-CH₂F-CH=CH-CHF₂, (E)-CH₂F-CH=CH-CHF₂, (Z)-CH₃-CH=CH-CHF₂, (E)-CH₃-CH=CH-CHF₂, (Z)-CH₃-CH=CF-CH₂F, (E)-CH₃-CH=CF-CH₂F, (Z)-CH₃-CF=CH-CH₂F, (E)-CH₃-CF=CH-CH₂F, (Z)-CH₃-CF=CF-CH₃, (E)-CH₃-CF=CF-CH₃, (Z)-CH₂F-CH=CH-CH₂F, (E)-CH₂F-CH=CH-CH₂F, (Z)-CH₃-CH=CH-CH₂F, (E)-CH₃-CH=CH-CH₂F, (Z)-CH₃-CH=CF-CH₃, and (E)-CH₃-CH=CF-CH₃.

These fluoro-2-butenes may be used singly or in combination of two or more of them. The above fluoro-2-butenes include E/Z-geometric isomers as described above, and any fluoro-2-butene in each geometric isomer form can be used in the method for storing a fluoro-2-butene pertaining to the present embodiment.

When a fluoro-2-butene is stored in a container, a gas consisting only of the fluoro-2-butene may be stored in a container, or a mixed gas containing the fluoro-2-butene and a dilution gas may be stored in a container. As the dilution gas, at least one gas selected from nitrogen gas (N₂), helium (He), neon (Ne), argon (Ar), krypton (Kr), and xenon (Xe) can be used. The content of the dilution gas is preferably 90% by volume or less and more preferably 50% by volume or less relative to the total volume of the gases stored in a container.

### [Container]

The container in which a fluoro-2-butene is stored may be any container that can store a fluoro-2-butene and be sealed, and the shape, the size, the material, and the like are not specifically limited. The material of the container may be, for example, a metal, ceramics, or a resin. Examples of the metal include manganese steel, stainless steel, Hastelloy (registered trademark), and Inconel (registered trademark).

### [Metal impurity]

The fluoro-2-butene pertaining to the present embodiment contains or does not contain at least one of chromium, molybdenum, iron, zinc, and aluminum as a metal impurity. The fluoro-2-butene is stored in a container in which the total concentration of chromium, molybdenum, iron, zinc, and aluminum is 1,000 ppb by mass or less when containing at least one of chromium, molybdenum, iron, zinc, and aluminum. As described above, this condition suppresses the isomerization reaction of a fluoro-2-butene, and consequently, the fluoro-2-butene is unlikely to be isomerized during storage. In the description, the not containing something means that it cannot be quantified by using an inductively coupled plasma mass spectrometer (ICP-MS).

To suppress isomerization reaction of a fluoro-2-butene during storage, the total concentration of chromium, molybdenum, iron, zinc, and aluminum in the fluoro-2-butene is required to be 1,000 ppb by mass or less, but is preferably 500 ppb by mass or less and more preferably 100 ppb by mass or less.

To better suppress isomerization reaction of a fluoro-2-butene during storage, each concentration of chromium, molybdenum, iron, zinc, and aluminum in the fluoro-2-butene is preferably 300 ppb by mass or less and more preferably 100 ppb by mass or less. The total concentration of chromium, molybdenum, iron, zinc, and aluminum in a fluoro-2-butene is as described above.

The total concentration of chromium, molybdenum, iron, zinc, and aluminum in a fluoro-2-butene may be 1 ppb by mass or more.

The concentration of a metal impurity such as chromium, molybdenum, iron, zinc, and aluminum in a fluoro-2-butene may be quantified by using an inductively coupled plasma mass spectrometer (ICP-MS).

The above metal impurities may be contained as an elemental metal, a metallic compound, a metal halide, or a metal complex in a fluoro-2-butene. Of them, the metal halide is known to further accelerate isomerization reaction. A trace amount of a hydrogen halide and a metal contained in a fluoro-2-butene may be reacted in a container in which the fluoro-2-butene is stored, and a metal halide may be formed.

Examples of the form of the metal impurity in a fluoro-2-butene include microparticles, droplets, and gas. Chromium, molybdenum, iron, zinc, and aluminum are mixed in a fluoro-2-butene supposedly from a material, a reaction vessel, a refiner, or the like used to synthesize the fluoro-2-butene.

### [Method for producing fluoro-2-butene containing metal impurity at low concentration]

A fluoro-2-butene containing a metal impurity at a low concentration may be produced by any method, and examples of the method include a method of removing metal impurities from a fluoro-2-butene containing metal impurities at high concentrations. Metal impurities may be removed from a fluoro-2-butene by any method, and a known method may be used. Examples of the method include a method using a filter, a method using an adsorbent, and distillation.

The material of the filter through which a fluoro-2-butene gas selectively passes is preferably a resin and specifically preferably polytetrafluoroethylene to prevent a metal component from mixing with a fluoro-2-butene. The average pore size of the filter is preferably 0.01 um or more and 30 um or less and more preferably 0.1 um or more and 10 um or less. A filter having an average pore size within the above range can be used to thoroughly remove metal impurities and can allow a fluoro-2-butene gas to pass through at a sufficient flow rate, achieving high productivity.

The flow rate of a fluoro-2-butene gas passing through the filter is preferably 100 mL/min or more and 5,000 mL/min or less and more preferably 300 mL/min or more and 1,000 mL/min or less. The linear velocity of a fluoro-2-butene gas passing through the filter is preferably 3 m/hr or more and 150 m/hr or less and more preferably 9 m/hr or more and 30 m/hr or less. When the flow rate and the linear velocity of a fluoro-2-butene gas are within the above range, the fluoro-2-butene gas is prevented from reaching a high pressure, and this can reduce the risk of fluoro-2-butene gas leakage. In addition, high productivity can be achieved.

### [Pressure conditions during storage]

Pressure conditions during storage in the method for storing a fluoro-2-butene pertaining to the present embodiment are not specifically limited as long as a fluoro-2-butene can be sealed and stored in a container, but the pressure is preferably 0.05 MPa or more and 5 MPa or less and more preferably 0.1 MPa or more and 3 MPa or less. When the pressure conditions are within the above range, a fluoro-2-butene can be allowed to pass without warming through a container that is connected to a dry etching system.

### [Temperature conditions during storage]

Temperature conditions during storage in the method for storing a fluoro-2-butene pertaining to the present embodiment are not specifically limited, but the temperature is preferably -20°C or more and 50°C or less and more preferably 0°C or more and 40°C or less. At a temperature of -20°C or more during storage, a container is unlikely to deform and thus is unlikely to lose the airtightness. This reduces the possibility of oxygen, water, or the like entering the container. If oxygen, water, or the like entered a container, polymerization reaction or decomposition reaction of a fluoro-2-butene could be accelerated. At a temperature of 50°C or less during storage, polymerization reaction or decomposition reaction of a fluoro-2-butene is suppressed.

### [Etching]

The fluoro-2-butene pertaining to the present embodiment can be used as an etching gas. When an etching gas containing the fluoro-2-butene pertaining to the present embodiment is used in an etching process for producing a semiconductor having a film containing silicon (Si), a protective film is formed on a mask or a side wall, and thus etching selectivity is improved.

An etching gas containing the fluoro-2-butene pertaining to the present embodiment can be used in both plasma etching with plasma and plasmaless etching without plasma.

Examples of the plasma etching include reactive ion etching (RIE), inductively coupled plasma (ICP) etching, capacitively coupled plasma (CCP) etching, electron cyclotron resonance (ECR) plasma etching, and microwave plasma etching.

In plasma etching, plasma may be generated in a chamber in which a member to be etched is placed, or a plasma generation chamber may be installed separately from a chamber in which a member to be etched is placed (i.e., remote plasma may be used).

### [Examples]

The present invention will next be described more specifically with reference to examples and comparative examples. Fluoro-2-butenes containing metal impurities at various concentrations were prepared. Fluoro-2-butene preparation examples will be described below.

### (Preparation Example 1)

A manganese steel tank having a volume of 10 L and four manganese steel cylinders each having a volume of 1 L were prepared. These cylinders are called cylinder A, cylinder B, cylinder C, and cylinder D. The tank was filled with 5,000 g of (Z)-1,1,1,4,4,4-hexafluoro-2-butene (boiling point: 33°C) and was cooled at 10°C for liquefaction, and a liquid phase portion and a gas phase portion were formed at about 100 kPa. The cylinders A, B, C, and D were depressurized to 1 kPa or less by using a vacuum pump and then were cooled to -78°C.

From the upper outlet where the gas phase portion was present in the tank, 500 g of (Z)-1,1,1,4,4,4-hexafluoro-2-butene gas was extracted, allowed to pass through a filter, and collected in the cylinder A at a reduced pressure. The filter was a PTFE filter manufactured by Flon Industry and having an outer diameter of 50 mm, a thickness of 80 um, and an average pore size of 0.3 um. The flow rate of the gas passing through the filter was adjusted to 500 mL/min by using a mass flow controller. The amount of (Z)-1,1,1,4,4,4-hexafluoro-2-butene gas collected in the cylinder A was 491g.

The (Z)-1,1,1,4,4,4-hexafluoro-2-butene collected in the cylinder A is regarded as sample 1-1. The (Z)-1,1,1,4,4,4-hexafluoro-2-butene gas collected in the cylinder A was extracted from the upper outlet, and the concentrations of various metal impurities were determined by using an inductively coupled plasma mass spectrometer. The results are shown in Table 1. Conditions of inductively coupled plasma mass spectrometry were as follows:
Inductively coupled plasma mass spectrometer: Agilent 7900 ICP-MS manufactured by Agilent Technologies
Plasma induction coil power: 1,600 W
Sampling depth: 8 mm
Carrier gas flow rate: 0.7 L/min
First ion lens applied voltage: +5.5 V
Second ion lens applied voltage: -100 V
Sample solution: nitric acid at a concentration of 20% by volume

**[Table 1]**

| | Cr | Mo | Fe | Zn | Al | Total |
|---|---|---|---|---|---|---|
| Sample 1-1 | 3 | 2 | 6 | 6 | 5 | 22 |
| Sample 1-2 | 21 | 19 | 24 | 25 | 22 | 111 |
| Sample 1-3 | 165 | 161 | 188 | 187 | 174 | 875 |
| Sample 1-4 | 311 | 307 | 322 | 324 | 321 | 1585 |

| | | | | | | |
|---|---|---|---|---|---|---|
| *) The unit of numerical values is ppb by mass. | | | | | | |

Next, the temperature of the cylinder A was raised to about 10°C, and a liquid phase portion and a gas phase portion were formed. From the upper outlet where the gas phase portion was present in the cylinder A, 100 g of (Z)-1,1,1,4,4,4-hexafluoro-2-butene gas was extracted and transferred to the cylinder B at a reduced pressure. From the tank, 10 g of (Z)-1,1,1,4,4,4-hexafluoro-2-butene gas was extracted and transferred to the cylinder B at a reduced pressure. The temperature of the cylinder B was then raised to room temperature and was allowed to stand for 24 hours. The (Z)-1,1,1,4,4,4-hexafluoro-2-butene after standing is regarded as sample 1-2. From the upper outlet where the gas phase portion was present in the cylinder B after standing, the (Z)-1,1,1,4,4,4-hexafluoro-2-butene gas was extracted, and the concentrations of various metal impurities were determined by using an inductively coupled plasma mass spectrometer. The results are shown in Table 1.

In a similar manner, from the upper outlet where the gas phase portion was present in the cylinder A, 100 g of (Z)-1,1,1,4,4,4-hexafluoro-2-butene gas was extracted and transferred to the cylinder C at a reduced pressure. From the tank, 100 g of (Z)-1,1,1,4,4,4-hexafluoro-2-butene gas was extracted and transferred to the cylinder C at a reduced pressure. The temperature of the cylinder C was then raised to room temperature and was allowed to stand for 24 hours. The (Z)-1,1,1,4,4,4-hexafluoro-2-butene after standing was regarded as sample 1-3. From the upper outlet where the gas phase portion was present in the cylinder C after standing, the (Z)-1,1,1,4,4,4-hexafluoro-2-butene gas was extracted, and the concentrations of various metal impurities were determined by using an inductively coupled plasma mass spectrometer. The results are shown in Table 1.

In a similar manner, from the upper outlet where the gas phase portion was present in the cylinder A, 100 g of (Z)-1,1,1,4,4,4-hexafluoro-2-butene gas was extracted and transferred to the cylinder D at a reduced pressure.
From the tank, 200 g of (Z)-1,1,1,4,4,4-hexafluoro-2-butene gas was extracted and transferred to the cylinder D at a reduced pressure. The temperature of the cylinder D was then raised to room temperature and was allowed to stand for 24 hours. The (Z)-1,1,1,4,4,4-hexafluoro-2-butene after standing was regarded as sample 1-4. From the upper outlet where the gas phase portion was present in the cylinder D after standing, the (Z)-1,1,1,4,4,4-hexafluoro-2-butene gas was extracted, and the concentrations of various metal impurities were determined by using an inductively coupled plasma mass spectrometer. The results are shown in Table 1.

### (Preparation Example 2)

The same procedure as in Preparation Example 1 was performed except that (E)-1,1,1,4,4,4-hexafluoro-2-butene was used as a fluoro-2-butene, and samples 2-1 to 2-4 were prepared. The concentrations of various metal impurities in each sample were determined by using an inductively coupled plasma mass spectrometer. The results are shown in Table 2.

**[Table 2]**

| | Cr | Mo | Fe | Zn | Al | Total |
|---|---|---|---|---|---|---|
| Sample 2-1 | 2 | 4 | 7 | 6 | 7 | 26 |
| Sample 2-2 | 17 | 21 | 28 | 24 | 29 | 119 |
| Sample 2-3 | 132 | 157 | 191 | 186 | 192 | 858 |
| Sample 2-4 | 289 | 307 | 337 | 326 | 335 | 1594 |

| | | | | | | |
|---|---|---|---|---|---|---|
| *) The unit of numerical values is ppb by mass. | | | | | | |

### (Preparation Example 3)

The same procedure as in Preparation Example 1 was performed except that (Z)-1,1,1,2,4,4,4-heptafluoro-2-butene was used as a fluoro-2-butene, and samples 3-1 to 3-4 were prepared. The concentrations of various metal impurities in each sample were determined by using an inductively coupled plasma mass spectrometer. The results are shown in Table 3.

**[Table 3]**

| | Cr | Mo | Fe | Zn | Al | Total |
|---|---|---|---|---|---|---|
| Sample 3-1 | 3 | 3 | 6 | 7 | 8 | 27 |
| Sample 3-2 | 12 | 14 | 25 | 29 | 31 | 111 |
| Sample 3-3 | 128 | 131 | 188 | 193 | 200 | 840 |
| Sample 3-4 | 280 | 289 | 311 | 331 | 347 | 1558 |

| | | | | | | |
|---|---|---|---|---|---|---|
| *) The unit of numerical values is ppb by mass. | | | | | | |

### (Preparation Example 4)

The same procedure as in Preparation Example 1 was performed except that (E)-1,1,1,2,4,4,4-heptafluoro-2-butene was used as a fluoro-2-butene, and samples 4-1 to 4-4 were prepared. The concentrations of various metal impurities in each sample were determined by using an inductively coupled plasma mass spectrometer. The results are shown in Table 4.

**[Table 4]**

| | Cr | Mo | Fe | Zn | Al | Total |
|---|---|---|---|---|---|---|
| Sample 4-1 | 5 | 3 | 7 | 7 | 8 | 30 |
| Sample 4-2 | 21 | 15 | 27 | 27 | 33 | 123 |
| Sample 4-3 | 138 | 128 | 192 | 190 | 202 | 850 |
| Sample 4-4 | 290 | 276 | 339 | 332 | 349 | 1586 |

| | | | | | | |
|---|---|---|---|---|---|---|
| *) The unit of numerical values is ppb by mass. | | | | | | |

### (Preparation Example 5)

The same procedure as in Preparation Example 1 was performed except that (Z)-1,1,1,2,3,4,4,4-octafluoro-2-butene was used as a fluoro-2-butene, and samples 5-1 to 5-4 were prepared. The concentrations of various metal impurities in each sample were determined by using an inductively coupled plasma mass spectrometer. The results are shown in Table 5.

**[Table 5]**

| | Cr | Mo | Fe | Zn | Al | Total |
|---|---|---|---|---|---|---|
| Sample 5-1 | 7 | 6 | 9 | 6 | 9 | 37 |
| Sample 5-2 | 26 | 24 | 35 | 21 | 34 | 140 |
| Sample 5-3 | 144 | 137 | 210 | 184 | 203 | 878 |
| Sample 5-4 | 302 | 300 | 353 | 318 | 350 | 1623 |

| | | | | | | |
|---|---|---|---|---|---|---|
| *) The unit of numerical values is ppb by mass. | | | | | | |

### (Preparation Example 6)

The same procedure as in Preparation Example 1 was performed except that (E)-1,1,1,2,3,4,4,4-octafluoro-2-butene was used as a fluoro-2-butene, and samples 6-1 to 6-4 were prepared. The concentrations of various metal impurities in each sample were determined by using an inductively coupled plasma mass spectrometer. The results are shown in Table 6.

**[Table 6]**

| | Cr | Mo | Fe | Zn | Al | Total |
|---|---|---|---|---|---|---|
| Sample 6-1 | 5 | 6 | 6 | 8 | 8 | 33 |
| Sample 6-2 | 21 | 25 | 27 | 32 | 31 | 136 |
| Sample 6-3 | 138 | 138 | 153 | 202 | 201 | 832 |
| Sample 6-4 | 288 | 301 | 312 | 322 | 336 | 1559 |

| | | | | | | |
|---|---|---|---|---|---|---|
| *) The unit of numerical values is ppb by mass. | | | | | | |

### (Example 1)

The cylinder A was allowed to stand at 20°C for 30 days, and then from the gas phase portion of the cylinder A, (Z)-1,1,1,4,4,4-hexafluoro-2-butene gas was extracted and analyzed by gas chromatography to quantify the concentration of (E)-1,1,1,4,4,4-hexafluoro-2-butene in sample 1-1. As a result, (E)-1,1,1,4,4,4-hexafluoro-2-butene, which is the isomerization reaction product of (Z)-1,1,1,4,4,4-hexafluoro-2-butene, was not detected.

Measurement conditions of the gas chromatography were as follows:
Gas chromatograph: GC-2014 manufactured by Shimadzu Corporation
Column: CarbopackB phase1% sp-1000
Injection temperature: 200°C
Column temperature: 150°C
Detector: FID
Detector temperature: 200°C
Carrier gas: helium
Detection limit: 1 ppm by mass

### (Examples 2 to 18 and Comparative Examples 1 to 6)

Analysis objects and analysis results in Examples 2 to 18 and Comparative Examples 1 to 6 are listed in Table 7 in comparison with those in Example 1. In other words, substantially the same analysis as in Example 1 was performed except the items listed in Table 7.

**[Table 7]**

| | Sample/cylinder | Fluoro-2-butene | Geometric isomer concentration (% by mass) |
|---|---|---|---|
| Ex. 1 | 1-1/A | (Z)-1,1,1,4,4,4-Hexafluoro-2-butene | Not detected |
| Ex. 2 | 1-2/B | Ditto | Not detected |
| Ex. 3 | 1-3/C | Ditto | Not detected |
| Comp. Ex. 1 | 1-4/D | Ditto | 2 |
| Ex. 4 | 2-1/A | (E)-1,1,1,4,4,4-Hexafluoro-2-butene | Not detected |
| Ex. 5 | 2-2/B | Ditto | Not detected |
| Ex. 6 | 2-3/C | Ditto | Not detected |
| Comp. Ex. 2 | 2-4/D | Ditto | 5 |
| Ex. 7 | 3-1/A | (Z)-1,1,1,2,4,4,4-Heptafluoro-2-butene | Not detected |
| Ex. 8 | 3-2/B | Ditto | Not detected |
| Ex. 9 | 3-3/C | Ditto | Not detected |
| Comp. Ex. 3 | 3-4/D | Ditto | 2 |
| Ex. 10 | 4-1/A | (E)-1,1,1,2,4,4,4-Heptafluoro-2-butene | Not detected |
| Ex. 11 | 4-2/B | Ditto | Not detected |
| Ex. 12 | 4-3/C | Ditto | Not detected |
| Comp. Ex. 4 | 4-4/D | Ditto | 6 |
| Ex. 13 | 5-1/A | (Z)-1,1,1,2,3,4,4,4-Octafluoro-2-butene | Not detected |
| Ex. 14 | 5-2/B | Ditto | Not detected |
| Ex. 15 | 5-3/C | Ditto | Not detected |
| Comp. Ex. 5 | 5-4/D | Ditto | 3 |
| Ex. 16 | 6-1/A | (E)-1,1,1,2,3,4,4,4-Octafluoro-2-butene | Not detected |
| Ex. 17 | 6-2/B | Ditto | Not detected |
| Ex. 18 | 6-3/C | Ditto | Not detected |
| Comp. Ex. 6 | 6-4/D | Ditto | 5 |

## Claims

1. A method for storing a fluoro-2-butene represented by general formula C₄HₓF_{y} where x is 0 or more and 7 or less, y is 1 or more and 8 or less, and x + y is 8, wherein
the fluoro-2-butene contains or does not contain at least one of chromium, molybdenum, iron, zinc, and aluminum as a metal impurity, and the fluoro-2-butene is stored in a container in which a total concentration of chromium, molybdenum, iron, zinc, and aluminum is 1,000 ppb by mass or less when the fluoro-2-butene contains at least one of chromium, molybdenum, iron, zinc, and aluminum.

2. The method for storing a fluoro-2-butene according to claim 1, wherein the fluoro-2-butene is at least one selected from (Z)-1,1,1,4,4,4-hexafluoro-2-butene, (E)-1,1,1,4,4,4-hexafluoro-2-butene, (Z)-1,1,1,2,4,4,4-heptafluoro-2-butene, (E)-1,1,1,2,4,4,4-heptafluoro-2-butene, (Z)-1,1,1,2,3,4,4,4-octafluoro-2-butene, and (E)-1,1,1,2,3,4,4,4-octafluoro-2-butene.

3. The method for storing a fluoro-2-butene according to claim 1 or claim 2, wherein the fluoro-2-butene is stored at a temperature of -20°C or more and 50°C or less.
